Europäisches Patentamt

European Patent Office

Office européen des brevets

(19)

(11) Veröffentlichungsnummer : **0 435 913 B1**

(12)

# EUROPÄISCHE PATENTSCHRIFT

(45) Veröffentlichungstag der Patentschrift :
17.03.93 Patentblatt 93/11

(51) Int. Cl.⁵ : **A61K 35/78, A61K 9/46**

(21) Anmeldenummer : 89910611.6

(22) Anmeldetag : 19.09.89

(86) Internationale Anmeldenummer :
PCT/EP89/01087

(87) Internationale Veröffentlichungsnummer :
WO 90/03179 05.04.90 Gazette 90/08

(54) **BRAUSETABLETTE.**

(30) Priorität : 22.09.88 DE 3822277

(43) Veröffentlichungstag der Anmeldung :
10.07.91 Patentblatt 91/28

(45) Bekanntmachung des Hinweises auf die
Patenterteilung :
17.03.93 Patentblatt 93/11

(84) Benannte Vertragsstaaten :
AT BE CH DE FR GB IT LI LU NL SE

(56) Entgegenhaltungen :
AU-B- 511 852
FR-M- 7 749
Rote Liste, 1988, Editio Cantor, (Aulendorf/-
Württ., DE), Zusammenfassung Nr. 05031,
"Zeller-Kopfschmerz-Dragees"
Dictionnaire Vidal, 1988, 64. Auflage, OVP, (Paris, FR) Seite 145, "Aspirine UPSA vitaminée C
tamponnée effervescente"

(73) Patentinhaber : Stephan, Günter
Happoldstrasse 47
W-7000 Stuttgart 30 (DE)

(72) Erfinder : STEPHAN, Günter
Happoldstr. 47
W-7000 Stuttgart 30 (DE)
Erfinder : STEPHAN, Dieter
Gehrenwaldstr. 35 A
W-7000 Stuttgart 60 (DE)
Erfinder : HONERLAGEN, Hans
Binsenmatt 2
CH-6314 Unterägri (CH)
Erfinder : MITSCHKA, Jochen
Strünkerhof 13a
W-5302 Much (DE)

(74) Vertreter : Patentanwälte Wenzel & Kalkoff
Grubes Allee 26 Postfach 730466
W-2000 Hamburg 73 (DE)

EP 0 435 913 B1

Jouve, 18, rue Saint-Denis, 75001 PARIS

## Beschreibung

Die Erfindung betrifft eine analgetische Tablette mit einem Extrakt des Typs Extrakt Salicis (Weidenrindenextrakt) als Wirkstoff.

Bekannte Tabletten dieser Art (s. Rote Liste, Zus.-Nr. 05031, Zeller-Kopfschmerz-Dragées) werden vom Verkehr jedoch aufgrund der umständlichen, vielen Menschen Unbehagen bereitenden Darreichungsform als zu schluckende Tablette weniger akzeptiert und daher oftmals gemieden. Deshalb werden schon seit längerer Zeit Brausetabletten im pharmazeutischen Bereich als Träger für wasserlösliche und/oder hygroskopische Mittel zu deren Verabreichung in flüssiger Form vielfältig verwendet. Eines der Hauptanwendungsgebiete ist die Verabreichung von relativ hohen Dosen von Vitamin- und Mineralpräparaten. Die Brausetablette stellt, wie allgemein bekannt, eine galenische Form dar, um einen Wirkstoff in einer schnell vom menschlichen Körper zu resorbierenden Form anzubieten.

Es ist zwar allgemein bekannt (s. Fachveröffentlichungen Acta Facultatis Pharmaceuticae, TOM XXVII, 1975, S. 7 bis 24, bes. S. 12 und 18, sowie Helwig "Moderne Arzneimitteln", Wiss Verlagsgesellschaft, Stuttgart, S. 20/21 unter "Aspirin® plus C" und "Boxacin®"), Brausetabletten unter Verwendung von auf synthetischem Wege gewonnenen Analgetika herzustellen. Dabei müssen aber zwei maßgebliche negative Eigenschaften bzw. Wirkungen synthetisch hergestellter Salicylsäure in Kauf genommen werden, nämlich

a) die Reizung der (Magen-)Schleimhäute und

b) die Hemmung der Thrombozytenaggregation (Herabsetzung der Blutgerinnungsfähigkeit).

Darüber hinaus sind heute ganz generell Pharmazeutika auf der Basis von Acetylsalicylsäure (ASS) bekannt, die üblicherweise als Tabletten oder Pulver verabreicht werden. Diese weisen jedoch wie die Salicylsäure-Brausetabletten einen strengen Geschmack und einen relativ hohen pH-Wert auf.

Hauptziel der Erfindung ist es, für die bekannten, eingangs bezeichneten analgetischen Tabletten unter Beseitigung deren dargelegter Nachteile eine neue, heute vom Verkehr eher akzeptierte galenische Verabreichungsform zu schaffen.

Diese Ziel wird nach der Erfindung dadurch erreicht, daß der Extrakt als wasserlösliche Substanz in einem Gemisch mit einem ebenfalls gut wasserlöslichen, Gas entwickelten und freisetzenden, ein Bindemittel enthaltenden Trägerstoff sowie mit Aromastoffen in der galenischen Form einer Brausetablette verpreßt ist. Somit ist die Konfektionierung eines Extrakt Salicis-Wirkstoffes als Brausetablette ermöglicht, wobei die als Brausetablette vorliegende analgetische Tablette vom Verkehr heute besonders akzeptiert wird. Dazu ist die Wasserlöslichkeit sowohl des Extraktes als auch des Trägerstoffes gewährleistet, wobei der Trägerstoff, um eine unter verbrauchsüblichen Gesichtspunkten akzeptable Brausetablette zu bilden, noch verschiedene Hilfsstoffe enthält. Um den Efferveszenz-Charakter in Verbindung mit dem in einer vernünftigen Zeit erfolgenden Auflösen der Tablette und andererseits eine hinreichende Bindung des Gemisches zur Herstellung und Transportsowie Lagerfähigkeit der gewünschten galenischen Form (Tablette) sicherzustellen, aber gleichzeitig auch die Verabreichung an den Patienten möglichst angenehm zu machen, liegen die Hilfsstoffe in einem Gemisch mit dem Trägerstoff vor, das einerseits gut wasserlöslich ist sowie Gas entwickelt und freisetzt und andererseits auch Bindemittel enthält sowie zudem mit Aromastoffen zur Geschmacksverbesserung versetzt ist. Mit anderen Worten erreicht man insgesamt eine galenisch zweckmäßige Verabreichungsform der in der Regel außerordentlich hygroskopischen Extrakten des Typs Salicis (Weidenrindenextrakt), und zwar mit hohem Wirkstoffgehalt. Denn da Brausetabletten zur Einnahme aufgelöst werden, können in ihnen angesichts der Tatsache, daß ihre Größe keine oder allenfalls eine untergeordnete Rolle spielt, hohe Extrakt-/Wirkstoffmengen untergebracht werden. Dabei ist es von besonderer Bedeutung, daß man diese hohen Wirkstoffmengen äußerst schnell resorbierbar macht, also für den Patienten möglichst schnell den durch die Einnahme angestrebten Effekt herbeiführt (Erreichung des $C_{max}$-Zustandes). Durch die gleichmäßige schnelle Resorption relativ hoher Wirkstoffmengen ist es möglich, bei Langzeitbehandlung von Patienten über längere Zeit hinweg einen gleichmäßigen Wirkstoffspiegel zu erzeugen. Gleichzeitig erhält man eine von breitesten Käufer- bzw. Patientenschichten äußerst akzeptable Darreichungsform für Naturprodukte. Die maßgeblichen Unterschiede zwischen dem Extrakt Salicis bzw. dem Wirkstoff Salicin als Naturprodukt einerseits und der ASS (Acetylsalicylsäure) als synthetischem Stoff bestehen darin, daß das Salicin ein Glycosid ist, d.h. der Wirkstoff Salicylsäure ist an ein Zuckermolekül gebunden, das erst im Darm abgespalten wird, wo sich die angestrebte Salicylsäure als schmerzlindernder oder -hemmender Wirkstoff ausbildet. Bei der ASS erfolgt diese Bildung der Salicylsäure zwar auch im Darm, jedoch ist der Ausgangsstoff ASS in seiner chemischen Struktur ein völlig anderer; z.B. enthält ASS kein Zuckermolekül. Die zur Herbeiführung der analgetischen Wirkung erforderliche Menge von ASS ist größer, und zudem hat ASS durch seine freie Säuregruppe den erwähnten negativen, nämlich schleimhautreizenden Einfluß beim Durchgang durch den Magen.

Gegenüber den bekannten ASS-Schmerzmitteln bietet eine einen hochkonzentrierten Extrakt Salicis enthaltende Brausetablette nach der Erfindung damit insbesondere folgende Vorteile:

a. Bei dem Extract Salicis bzw. dem Wirkstoff Salicin ist zwar wie bei ASS Salicylsäure als terminaler Wirkstoff anzusehen, jedoch entsteht diese erst durch verschiedene Umwandlungen des Glycosids im Darm und Resorption in der Leber. Dadurch treten die für ASS typischen Nebenwirkungen an der Magenschleimhaut beim Extrakt Salicis infolge der Bildung der Salicylsäure in der Leber nicht auf, während durch die Umwandlung zwar die Wirkung des Extract Salicis später als bei ASS auftritt, dafür jedoch auch länger vorhält.

b. Der pH-Wert von Extract Salicis ist niedriger als bei ASS.

c. Gegenüber dem strengen Geschmack von ASS ist der Extract Salicis nur wenig bitter.

d. Beim Extract Salicis handelt es sich um ein Naturprodukt, und solche Naturprodukte finden gegenüber synthetisch hergestellten Pharmazeutika im Markt eine zunehmende Akzeptanz.

e. Eine Brausetablette stellt, da sie den Wirkstoff in einer schnell zu resorbierenden Form mit dem Ziel eines schnellen Wirkungseintritts anbietet, die optimale galenische Form für die beim Extract Salicis später als bei ASS eintretende Wirkung bei gleichzeitig längerem Anhalten der Wirkung dar, kombiniert also diese Vor- und Nachteile in idealer Form.

f. Da der Extract Salicis mit dem hohen Salicingehalt sehr hygroskopisch ist, bei Brausetabletten die Stoffe aber ebenfalls diese Eigenschaft haben, stellt eine Brausetablette hierfür eine bevorzugte Trägerform dar.

Vorteilhaft kann der Extract Salicis etwa 3 bis 20 Gew.% Salicin, vorzugsweise 7,5 Gew.%, enthalten. Damit ist es möglich, das Salicin wesentlich niedriger zu dosieren. So muß man bei ASS die Drogenmenge zur Erzielung der angestrebten schmerzlindernden Wirkung mit Dosen von 250 bis 500 mg vorsehen, während das Salicin mit 40 bis 120 mg bemessen werden kann.

Es ist weiterhin möglich, daß die Brausetablette zusätzlich einen wasserlöslichen Bestandteil eines Vitamin C-Produktes, vorzugsweise einen Extrakt aus Acerola und Zitrone enthält, so daß zusätzlich zu der primär angestrebten schmerzlindernden Wirkung dem Körper weitere Abwehrstoffe in pharmakologisch sinnvoller Weise zugeführt werden können.

Die insgesamt mit der Erfindung erzielbaren Vorteile sind nach allem darin zu sehen, daß durch die Wahl von Brausetabletten als Träger für Naturprodukte einerseits die Möglichkeit eines Ersatzes von synthetischen Arzneimitteln in einer besonders akzeptablen Darreichungsform geschaffen wird, während andererseits Nebenwirkungen solcher synthetischen Mittel durch Verlagerung der Umwandlungsprozesse an andere Stellen der menschlichen Physis ausgeschaltet und längere bzw. dauerhaftere Wirkungen erzielt werden, was wiederum die Dosierungserfordernisse senkt. Mit speziellem Blick auf ASS-Erzeugnisse ist weiter hervorzuheben, daß aufgrund mehrerer Studien festgestellt wurde (s. z.B. Medizinische Monatsschrift für Pharmazeuten, 7/1988, S. 230/2; Pharmazeutische Zeitung, Nr. 15, 1988, S. 9-13), daß regelmäßige Gaben solcher Erzeugnisse das Herzinfarktrisiko um 47 % vermindern, wobei jedoch die Tests bei ungefähr 30 % der Probanden wegen Problemen im Magen- und Darmtrakt abgebrochen werden mußten. Diesen Nachteil beseitigt die Verwendung des Weidenrindenextraktes dadurch, daß, wie erwähnt, die Salicylsäure erst in der Leber gebildet wird.

Ausführungsbeispiel:

Hergestellt wurden mehrere Chargen Brausetabletten mit folgender Rezeptur:

```
Extr. Salicis sicc.                        800 mg
Natriumhydrogencarbonat                    900 - 1050 mg
Citronensäure wasserfrei                  1650 - 1750 mg
Polyäthylenglykol 6000                     480 -  520 mg
Süßstoff (Aspartame, Cyclamat)              60 -   80 mg
Citronenaroma                               60 -   80 mg
Polyvinylpyrrolidon                        200 -  250 mg


Durchschnittliches Gesamtgewicht je Tablette   4400 mg
```

| Aussehen: | Beige-braune, runde Tabletten |
| Dicke: | 6,4 - 6,8 mm |
| Härte, kg: | 2,5 - 4 |
| Auflösezeit: | unter 7 Minuten |
| Geschmack: | säuerlich |
| Aussehen der Lösung: | leicht trübe, schaumig |
| Gehalt an Salicin: | 60 mg/Tablette |

Herstellungsgang:

1. Alle Wirk- und Hilfstoffe werden durch ein Freiwittsieb mit einer Maschenweite von 1,5 mm gesiebt
2. Süßstoff, Aromastoff und Polyvinylpyrrolidon werden in einem Labormischer homogen vermischt.
3. Extr. Salicis, Natriumhydrogencarbonat, Citronensäure und Polyäthylenglykol 6000 werden abgewogen und in einen Rhönradmischer gegeben. Nun wird die Mischung aus 2. zugegeben und 15 Minuten gemischt. Die Homogenität der Mischung wird visuell geprüft.
4. Aus der Mischung werden direkt die Tabletten gepreßt.

## Patentansprüche

1. Analgetische Tablette mit einem Extrakt des Typs Extrakt Salicis (Weidenrindenextrakt) als Wirkstoff, **dadurch gekennzeichnet,** daß der Extrakt als wasserlösliche Substanz in einem Gemisch mit einem ebenfalls gut wasserlöslichen, Gas entwickelnden und freisetzenden, ein Bindemittel enthaltenden Trägerstoff sowie mit Aromastoffen in der galenischen Form einer Brausetablette verpreßt ist.

2. Analgetische Tablette nach Anspruch 1, **dadurch gekennzeichnet,** daß der Extrakt Salicis etwa 3 bis 20 Gew.% Salicin enthält.

3. Analgetische Tablette nach Anspruch 1 oder 2, **dadurch gekennzeichnet,** daß der Extrakt Salicis 7,5 Gew.% Salicin enthält.

4. Analgetische Tablette nach einem der Ansprüche 1 bis 3, **dadurch gekennzeichnet,** daß sie zusätzlich einen wasserlöslichen Bestandteil eines Vitamin-C-Produktes enthält.

5. Analgetische Tablette nach Anspruch 4, **dadurch gekennzeichnet,** daß der Vitamin-C-Bestandteil ein Extrakt aus Acerola und Zitrone ist.

## Claims

1. Analgesic tablet with an extract of the salicis extract type (willow bark extract) as active substance, **characterized in** that the extract is compressed, in the galenic form of an effervescent tablet, as a water-soluble substance in a mixture with an also easily water-soluble, gas developing and releasing carrier substance containing a binding agent, as well as with aromatic substances.

2. Analgesic tablet as claimed in claim 1, **characterized in** that the salicis extract contains approximately 3 to 20% by weight of salicin.

3. Analgesic tablet as claimed in claim 1 or 2, **characterized in** that the salicis extract contains 7,5% by weight of salicin.

4. Analgesic tablet as claimed in any one of claims 1 to 3, **characterized in** that the tablet additionally contains a water-soluble component of a vitamin C product.

5. Analgesic tablet as claimed in claim 4, **characterized in** that the vitamin C component is an extract of Acerola and lemon.

**Revendications**

1. Comprimé analgésique comportant un extrait du type extrait de salicis (extrait d'écorce de saule) comme substance active, caractérisé en ce que l'extrait est comprimé, sous la forme galénique d'un comprimé effervescent, comme une substance soluble dans l'eau dans un mélange comportant également une substance porteuse facilement soluble dans l'eau développant et générant du gaz contenant un agent de liaison, de même que des substances aromatiques.

2. Comprimé analgésique selon la revendication 1, caractérisé en ce que l'extrait salicis utilisé comprend approximativement 3 à 20 % en poids de salicine.

3. Comprimé analgésique selon la revendication 1 ou la revendication 2, caractérisé en ce que l'extrait de salicis utilisé contient 7,5 % en poids de salicine.

4. Comprimé analgésique selon l'une quelconque des revendications 1 à 3, caractérisé en ce qu'il comporte en outre un composant soluble dans l'eau d'un produit du type vitamine C.

5. Comprimé analgésique selon la revendication 4, caractérisé en ce que le composant de vitamine C est un extrait d'Acerola et de citron.